# EUROPEAN PATENT SPECIFICATION

(11) **EP 1 928 413 B1**
(45) Date of publication and mention of the grant of the patent: **05.05.2010**
(21) Application number: 06798894.9
(22) Date of filing: 25.09.2006
(51) Int. Cl.: A61K 9/08, A61K 47/02, A61K 47/10, A61K 47/26, A61K 47/18

(54) **HFSH AQUEOUS FORMULATION**
WÄSSRIGE HFSH-FORMULIERUNG
FORMULATION AQUEUSE DE hFSH

(30) Priority: 27.09.2005 KR 20050089853
(43) Date of publication of application: 11.06.2008
(73) Proprietor: LG Life Sciences Ltd., Seoul 150-721 (KR)
(72) Inventor: CHOI, Suk Young, Daejeon 305-752 (KR); JEH, Hoon Sung, Daejeon 305-350 (KR)
(74) Representative: Gillard, Richard Edward
(86) International application number: PCT/KR2006/003811
(87) International publication number: WO 2007/037607

(56) References cited:
- WO-A-2004/087213
- WO-A1-00/04913
- WO-A2-03/039485
- US-A- 5 929 028
- US-A1- 2004 028 733

## Description

### Technical Field

The present invention relates to an aqueous formulation of a human follicle stimulating hormone (hFSH) which is stabilized to maintain the activity of hFSH for a prolonged period of time. More specifically, the present invention relates to an hFSH aqueous formulation comprising a therapeutically effective amount of hFSH stabilized in a phosphate buffer containing glycine, a non-ionic surfactant and methionine.

### Background Art

A human follicle stimulating hormone (hFSH) is a reproduction-related hormone which plays an essential role in the reproductive functions, in conjunction with Luteinizing Hormone (LH) and human Chorionic Gonadotropin (hCG). Structurally, hFSH is a heterodimeric glycoprotein formed by the non-covalent association of alpha and beta subunits. Alpha-subunit consists of 92 amino acid residues while beta-subunit consists of 111 amino acid residues, each of which retains two Asn-linked glycosylation sites (Human Reproduction Update 1998, Vol 4, No. 6 pp 862-881).

Until the 1980's, a primary source of hFSH was urine-derived FSH isolated from urine of child-bearing aged women. A further purified form of high-purity, urine-derived FSH was introduced in the 1990's, and finally a recombinant FSH was developed and widely used since the year of 1998. FSH is secreted by the pituitary gland. FSH stimulates the growth and maturation of the ovarian follicles in females. Therefore, FSH is a critical hormone responsible for ovulation of females and is used in the infertility treatment of anovulatory infertile women and Ovum Aspiration/Ovum Pickup (OPU) for artificial insemination.

Generally, proteins have a very short half-life, and undergo denaturation such as aggregation of monomers, dissociation of dimers, and adsorption on the surfaces of vessels, upon exposure to various factors such as unfavorable temperatures for the expression of activity of proteins, water-air interface, high pressure, physical/ mechanical stress, organic solvents and microbial contamination. Consequently, the denatured proteins lose intrinsic physicochemical properties and physiologically active effects. Denaturation of proteins is irreversible and therefore proteins, once denatured, can hardly recover their native properties to the initial state.

In particular, proteins which have a heterodimeric structure consisting of two different subunits and are administered in a trace amount of less than several hundred micrograms each time, such as hFSH, suffer from problems associated with a relatively high loss of proteins including the loss of proteins due to dissociation of dimers in aqueous solutions and adsorption of proteins on the inner surface of vessels. The dissociated proteins lose their own physiological activity, and the dissociated monomers are readily susceptible to aggregation. Further, the proteins adsorbed on the inner surface of vessels are also readily vulnerable to aggregation via the denaturation process. When they are administered into the human body, the thus-denatured proteins may serve as the cause of formation of antibodies against naturally-occurring proteins in the body. Therefore, the proteins of interest should be administered in the sufficiently stable state. To this end, a great deal of research has been made on the development of a method for preventing denaturation of proteins in the solution.

Some of protein pharmaceuticals have overcome stability problems via lyophilization (freeze-drying). As an example, US Patent No. 5270057 discloses a lyophilized formulation comprising gonadotropin (e.g. LH, TSH, FSH and hCG) stabilized with polycarboxylic acid or a salt thereof, preferably citric acid and a non-reducing disaccharide, sucrose. US Patent No. 5650390 discloses a lyophilized formulation comprising FSH, LH or hCG stabilized by means of a combination of sucrose and glycine. However, lyophilized products are inconvenient in that they must be dissolved in water for injection (WFI) for reconstitution prior to use thereof. Further, the production process of the lyophilized products involves a freeze-drying step, and therefore suffers from a need for a heavy investment such as use of a large-scale freeze dryer.

As an alternative measure to cope with such limitations, the stability of the protein may be improved by adding a stabilizer to the protein in solution state. As examples of useful protein stabilizers, there are known surfactants, serum albumin, polysaccharides, amino acids, polymers, salts and the like (John Geigert, J. Parenteral Sci. Tech., 43, No 5, 220-224, 1989; David Wong, Pharm. Tech., October, 34-48, 1997; and Wei Wang, Int. J. Pharm., 185, 129-188, 1999). However, the most suitable stabilizer should be selected and used taking into consideration unique physicochemical properties of individual proteins, and combined use of different protein stabilizers may bring about adverse side effects as opposed to expected effects, due to competitive action and adverse reaction between individual stabilizers. Further, successful stabilization of proteins present in the solution requires careful attention and many efforts, since the individual protein stabilizers have a specific concentration range favorable for stabilization of the corresponding proteins (Wei Wang, Int. J. Pharm. 185, 129-188, 1999).

Meanwhile, US Patent No. 5929028 discloses a stable liquid formulation of gonadotropin, which is prepared by dissolving gonadotropin (e.g. LH, TSH, FSH, and hCG) in a solution composed of a stabilizing amount of a polycarboxylic acid or a salt thereof, a stabilizing amount of a thioether compound, a non-reducing sugar and a non-ionic surfactant. According to this art, sodium citrate is described as the most preferred form of polycarboxylic acid or a salt thereof and the formulation contains 1.47% sodium citrate. An aqueous 1% citric acid solution exhibits strong acidity of pH 2.2 (Handbook of pharmaceutical excipients 2nd edition, p124). It is widely known in the art that citric acid or a salt thereof is an excipient inducing severe pain at the administration site upon injection thereof. For convenience of patients, those skilled in the art restrain from using citric acid or a salt thereof. The art described in US Patent No. 5929028 suffers from the problem of patient inconvenience due to pain of the drug administration site caused by inclusion of 1.47% sodium citrate in the pharmaceutical formulation.

US Patent No. 6706681 discloses a liquid pharmaceutical composition comprising hCG stabilized with a phosphate buffer (pH 7.0) containing a polyalcohol or a non-reducing sugar, preferably mannitol as a stabilizer. Applicability of this art is confined to hCG liquid formulations which are used at a high dose of more than 10,000 IU each time, and the test was not conducted for FSH liquid formulations which are used in a trace amount of 75 to 250 IU as a daily dose.

WO 2004/087213 discloses a liquid pharmaceutical composition comprising follicle-stimulating hormone (FSH, human or recombinant), methionine, a non-ionic surfactant selected from Pluronics (poloxamers), phosphate buffer at a pH of about 6.0-8.0 and *m*-cresol as bacteriostatic agent.

As can be seen from the above prior arts, there is an urgent need for the development of a novel liquid formulation which is capable of stably maintaining the activity of hFSH for an extended period of time.

### Disclosure of Invention

### Technical Problem

Therefore, the present invention has been made in view of the above problems, and it is an object of the present invention to provide an aqueous formulation of a human follicle stimulating hormone (hFSH) stabilized to maintain the activity of hFSH for a prolonged period of time.

### Technical Solution

Hereinafter, the present invention will be described in more detail.

In accordance with an aspect of the present invention, the above and other objects can be accomplished by the provision of an aqueous formulation of a human follicle stimulating hormone (hFSH), comprising a therapeutically effective amount of hFSH, and glycine, methionine, a non-ionic surfactant and a phosphate buffer as stabilizers.

That is, according to the formulation of the present invention, hFSH is stabilized in a phosphate buffer containing glycine, methionine and a non-ionic surfactant, such that the activity of hFSH can be maintained for a prolonged period of time.

### Best Mode for Carrying Out the Invention

As a source of hFSH, the aqueous formulation of the present invention may use the naturally-occurring hFSH in the body, or recombinant FSH (rFSH) which is expressed, isolated and purified from animal cells using recombinant technologies. Therefore, hFSH that can be used in the present invention encompasses all species of hFSH including natural and synthetic types.

The aqueous formulation of the present invention includes glycine as a component of the stabilizer. Glycine in solution state enables gathering of greater numbers of water molecules around hFSH, thereby further stabilizing the outermost hydrophilic amino acids among numerous amino acids constituting hFSH and consequently stabilizing hFSH (Wang, Int. J. Pharm. 185 (1999) 129-188). The content of glycine is in the range of 0.01 to 50% (w/v), preferably 0.1 to 10% (w/v), and more preferably 1 to 5% (w/v).

The formulation of the present invention includes methionine as another component of the stabilizer. Methionine serves to stabilize hFSH by preventing oxidation of hFSH in the aqueous solution (Wang, Int. J. Pharm. 185 (1999) 129-188). The content of methionine is in the range of 0.001 to 2% (w/v), preferably 0.01 to 1% (w/v), and more preferably 0.05 to 0.5% (w/v).

The formulation of the present invention also includes a non-ionic surfactant in order to prevent adsorption of hFSH on the surface of the vessel via stabilization thereof. Herein, the non-ionic surfactant lowers surface tension of a protein solution, thereby preventing adsorption or aggregation of the proteins on the hydrophobic surface. Preferred examples of the non-ionic surfactant that can be used in the present invention may include a polysorbate-based non-ionic surfactant and a poloxamer-based non-ionic surfactant. These non-ionic surfactants may be used alone or in any combination thereof. Particularly more preferred is the polysorbate-based non-ionic surfactant. Specific examples of the polysorbate-based non-ionic surfactant may include polysorbate 20, polysorbate 40, polysorbate 60 and polysorbate 80. More particularly preferred is polysorbate 20. The polysorbate 20 has a relatively low-critical micelle concentration. Therefore, the polysorbate 20 not only reduces or prevents surface adsorption of the proteins even at low concentrations, but also inhibits chemical degradation of the proteins. Use of high-concentration non-ionic surfactant in the aqueous formulation is not appropriate. This is because the use of the non-ionic surfactant at a high concentration results in interference effects, thus making it difficult to precisely evaluate the stability of the proteins, when the concentration determination or stability evaluation of the proteins is carried out using an analysis method such as UV-spectroscopy or Isoelectrical Focusing. Therefore, the aqueous formulation of the present invention contains the non-ionic surfactant in a low concentration of less than 0.1 % (w/v) and more preferably 0.001 to 0.02% (w/v).

The aqueous formulation of the present invention contains a phosphate buffer. A concentration of phosphates constituting the buffer solution is in the range of preferably 1 mM to 50 mM and more preferably 5 mM to 10 mM. The pH of the buffer solution is in the range of preferably 6.0 to 8.0 and more preferably 6.5 to 7.5.

In addition to the above-exemplified glycine, methionine, non-ionic surfactant and phosphate buffer, the formulation of the present invention may optionally include other components or materials known in the art, so long as they are not detrimental to desir ed effects of the present invention. For example, the formulation may further optionally include benzyl alcohol, cresol, alkyl paraben and the like, which are conventionally used as preservatives for protein pharmaceuticals.

Where appropriate, the formulation may further include an isotonic agent. Specific examples of the isotonic agent may include water-soluble inorganic salts such as sodium chloride and calcium chloride, and sugar alcohols such as mannitol, sorbitol, lactose, mannose, maltose, trehalose, glucose, glycerol, raffinose and sucrose. These materials may be used alone or in any combination thereof.

### Mode for the Invention

**EXAMPLES**

Now, the present invention will be described in more detail with reference to the following examples.

Examples 1 through 3: Preparation of hFSH aqueous formulations

Glycine, methionine and polysorbate 20 were added to a 10 mM phosphate solution to which hFSH was then added to a concentration of 150 IU/mL, thereby preparing an aqueous formulation of hFSH. 1 mL/vial of the thus-prepared solution was aliquoted into 3 mL glass vials which were then sealed and stored at 4°C and 25°C, respectively. The compositional formula of the aqueous formulations prepared in respective Examples is set forth in Table 1 below.

Comparative Example 1: Preparation of additive-free hFSH aqueous formulations

hFSH was added in a concentration of 150 IU/mL to a 10 mM phosphate solution. 1 mL/vial of the thus-prepared solution was aliquoted into 3 mL glass vials which were then sealed and stored at 4°C and 25°C, respectively.

**[Table 1]**

| | hFSH (IU/mL) | Glycine | Methionine | Polysorbate 20 | Phosphate buffer | pH |
|---|---|---|---|---|---|---|
| Ex. 1 | 150 | 20 | 0.5 | 0.1 | 10 mM | 7.0 |
| Ex. 2 | 150 | 20 | 1 | 0.1 | 10 mM | 7.0 |
| Ex. 3 | 150 | 20 | 1 | 0.2 | 10 mM | 7.0 |
| Comp. Ex. 1 | 150 | - | - | - | 10 mM | 7.0 |

| | | | | | | |
|---|---|---|---|---|---|---|
| *Unit: mg/mL | | | | | | |

Experimental Example 1: Stability test of hFSH aqueous formulations

After storage of the aqueous formulations of Examples 1 to 3 and Comparative Example 1 at 4°C for 0, 3 and 6 months and at 25°C for 0, 2, 4 and 6 months, % recovery of hFSH, % dissociation of hFSH into monomers and purity of hFSH were measured by SEC-HPLC, and % alpha-subunit oxidation of hFSH was measured by RP-HPLC, at the corresponding time points, respectively. The results thus obtained are respectively given in Tables 2 through 5 below.

**[Table 2]**

| | Recovery (%)* | | | | |
|---|---|---|---|---|---|
| | 4°C | | 25°C | | |
| | 3m** | 6 m | 2 m | 4 m | 6 m |
| Ex.1 | 97 | 105 | 97 | 100 | 103 |
| Ex. 2 | 98 | 105 | 97 | 100 | 103 |
| Ex.3 | 106 | 106 | 96 | 104 | 104 |
| Comp. Ex. 1 | 52 | 61 | 56 | 68 | 60 |

| | | | | | |
|---|---|---|---|---|---|
| * : Zero time based ** m: Month | | | | | |

**[Table 3]**

| | Dissociation (Monomer, %) | | | | |
|---|---|---|---|---|---|
| | 4°C | | 25°C | | |
| | 3m* | 6m | 2m | 4m | 6m |
| Ex. 1 | 2.6 | 2.2 | 2.7 | 3.5 | 3.5 |
| Ex. 2 | 2.6 | 2.2 | 2.7 | 3.1 | 3.6 |
| Ex. 3 | 5.5 | 2.1 | 2.9 | 4.4 | 4.4 |
| Comp. Ex. 1 | 11 | 12.6 | 7.4 | 9.1 | 11.8 |

| | | | | | |
|---|---|---|---|---|---|
| *m: Month | | | | | |

**[Table 4]**

| | Purity (%) | | | | |
|---|---|---|---|---|---|
| | 4°C | | 25°C | | |
| | 3m* | 6m | 2m | 4m | 6m |
| Ex.1 | 97.4 | 98.2 | 96.7 | 96.2 | 96.5 |
| Ex. 2 | 97.4 | 97.9 | 97.3 | 96.9 | 96.4 |
| Ex. 3 | 94.5 | 97.9 | 97.1 | 95.6 | 95.6 |
| Comp. Ex. 1 | 89 | 87.4 | 92.6 | 90.9 | 88.2 |

| | | | | | |
|---|---|---|---|---|---|
| *m: Month | | | | | |

As can be seen from Table 2, formulations of Examples 1 through 3 exhibited more than 95% recovery of hFSH under storage of the formulations at 4°C and 25°C for 6 months, whereas the formulation of Comparative Example 1 exhibited low % recovery (about 60%) of hFSH under the same conditions. In addition, as can be seen from Table 3, the formulations of Examples 1 through 3 exhibited less than 5% dissociation of hFSH into monomers under storage of the formulations at 4°C and 25°C for 6 months, whereas the formulation of Comparative Example 1 exhibited significantly increased % dissociation (more than 10%) of hFSH into monomers under the same conditions. Further, as can be seen from Table 4, the formulations of Examples 1 through 3 exhibited more than 95% purity of hFSH under storage of the formulations at 4°C and 25°C for 6 months, whereas the formulation of Comparative Example 1 exhibited a decreased purity (falling to 80%-ranges) of hFSH under the same conditions. From these results, it can be seen that the hFSH aqueous formulation comprising glycine, methionine and polysorbate 20 according to the present invention prevents the protein loss and denaturation due to adsorption of the protein on the inner surface of the vessel and prevents dissociation of the protein into the constituent monomers, and stabilizes hFSH for a prolonged period of time.

**[Table 5]**

| | Alpha-subunit oxidized form (%)* | | | | |
|---|---|---|---|---|---|
| | 4°C | | 254°C | | |
| | 3 m* | 6 m | 2 m | 4 m | 6 m |
| Ex.1 | 7.6 | 8.4 | 7.5 | 9.3 | 8.4 |
| Ex.2 | 8.2 | 7.6 | 7.5 | 8.6 | 7.7 |
| Ex.3 | 7.0 | 7.7 | 7.1 | 7.4 | 8.0 |
| Comp. Ex. 1 | 10.1 | 12.6 | 11.5 | 15.3 | 14.9 |

| | | | | | |
|---|---|---|---|---|---|
| *m: Month | | | | | |

As can be seen from Table 5, formulations of Examples 1 through 3 exhibited near 8%-ranges of alpha-subunit oxidation until 6 months upon storage of the formulations at 4°C and 25°C, whereas Comparative Example 1, i.e., an additive-free formulation, exhibited high % of alpha-subunit oxidation (about 15%), upon storage of the formulation at 25°C for 6 months. From these results, it can be seen that the aqueous formulations to which glycine, methionine and polysorbate 20 were added according to the present invention are stable aqueous formulations preventing oxidation of hFSH which may occur upon the long-term storage thereof.

Examples 4 and 5: Preparation of high-concentration hFSH aqueous formulations

Glycine, methionine, polysorbate 20, benzyl alcohol, sodium chloride and lactose were added to a 10 mM phosphate solution (pH 7.0) to which hFSH was then added to a concentration of 833 IU/mL, thereby preparing an aqueous formulation of hFSH. 0.5 mL/syringe of the thus-prepared solution was filled into 1 mL glass pre-filled syringes (Becton-Dickinson) which were then stored at 25°C. The compositional formula of the aqueous formulations thus prepared is set forth in Table 6 below.

Comparative Examples 2 through 4: Preparation of high-concentration hFSH aqueous formulations

Glycine, polysorbate 20 and benzyl alcohol were added to a 10 mM phosphate solution (pH 7.0) to which sodium chloride and lactose were then optionally added without addition of methionine, thereby preparing an aqueous formulation of hFSH. 0.5 mL/syringe of the thus-prepared solution was filled into 1 mL glass pre-filled syringes (Becton-Dickinson) which were then stored at 25°C. The compositional formula of the aqueous formulations thus prepared is set forth in Table 6 below.

**[Table 6]**

| | hFSH (IU/mL) | Glycine | Methionine | Polysorbate 20 | Benzyl alcohol | NaCl | Lactose |
|---|---|---|---|---|---|---|---|
| Ex. 4 | 833 | 14 | 0.5 | 0.1 | 10 | - | - |
| Ex.5 | 833 | 10 | 0.5 | 0.1 | 10 | - | 20 |
| Comp. Ex. 2 | 833 | 12 | - | 0.1 | 10 | 1 | - |
| Comp. Ex. 3 | 833 | 14 | - | 0.1 | 10 | - | - |
| Comp. Ex. 4 | 833 | 10 | - | 0.1 | 10 | - | 20 |

| | | | | | | | |
|---|---|---|---|---|---|---|---|
| * Unit: mg/mL | | | | | | | |

Experimental Example 2: Stability test of high-concentration hFSH aqueous formulations

After the storage of the aqueous formulations of Examples 4 and 5 and Comparative Examples 2 through 4 at 25°C for 0, 2, 4 and 6 months, % purity of hFSH was measured by SEC-HPLC, and % alpha-subunit oxidation of hFSH was measured by RP-HPLC, at the corresponding time points, respectively. The results thus obtained are respectively given in Table 7 below.

**[Table 7]**

| | Purity (%) | | | Alpha-subunit oxidized form (%) | | |
|---|---|---|---|---|---|---|
| | 2 m* | 4 m | 6 m | 2 m | 4 m | 6 m |
| Ex. 4 | 96.3 | 94.0 | 94.5 | 4.7 | 6.2 | 5.2 |
| Ex.5 | 94.8 | 92.8 | 92.9 | 5.3 | 7.3 | 7.6 |
| Comp. Ex. 2 | 96.6 | 92.6 | 94.4 | 12.3 | 16.4 | 17.6 |
| Comp. Ex. 3 | 96.5 | 94.2 | 94.3 | 12.8 | 18.1 | 19.0 |
| Comp. Ex. 4 | 95 | 93.4 | 92.9 | 22.5 | 35.2 | 45.9 |

| | | | | | | |
|---|---|---|---|---|---|---|
| *m: Month | | | | | | |

As can be seen from Table 7, formulations of Examples 4 and 5, which included glycine, methionine and polysorbate 20 as stabilizer components, exhibited more than 90% purity of hFSH until 6-month storage at room temperature (25°C), as measured by SEC-HPLC, and less than 10% alpha-subunit oxidation, as measured by RP-HPLC, thus representing that these formulations are hFSH-stabilized formulations. Further, addition of benzyl alcohol as a preservative had no significant effects on the stability of hFSH. Whereas, formulations of Comparative Examples 2 through 4 exhibited superior purity of more than 90%, but % alpha-subunit oxidation was high, significantly exceeding 10%, thus representing that these formulations are not stable.

### Industrial Applicability

As apparent from the above description, an aqueous formulation of the present invention can stably maintain the activity of a human follicle stimulating hormone (hFSH) for a prolonged period of time.

## Claims

1. An aqueous formulation of a human follicle stimulating hormone (hFSH), comprising a therapeutically effective amount of hFSH, and glycine, methionine, a non-ionic surfactant and a phosphate buffer as stabilizers.

2. The formulation according to claim 1, wherein the hFSH is a naturally-occurring hFSH or a recombinant FSH (rFSH).

3. The formulation according to claim 1, wherein the non-ionic surfactant is selected from the group consisting of a polysorbate-based non-ionic surfactant, a poloxamer-based non-ionic surfactant and any combination thereof.

4. The formulation according to claim 3, wherein the non-ionic surfactant is polysorbate 20.

5. The formulation according to claim 1, wherein glycine is included in an amount of 0.1 to 10% (w/v).

6. The formulation according to claim 1, wherein methionine is included in an amount of 0.01 to 2.0% (w/v).

7. The formulation according to claim 1, wherein the non-ionic surfactant is included in an amount of 0.001 to 0.02% (w/v).

8. The formulation according to claim 1, wherein the phosphate buffer has a salt concentration of 1 mM to 50 mM and a pH of 6.5 to 7.5.

9. The formulation according to claim 1, wherein the formulation further includes a preservative and/or an isotonic agent.

10. The formulation according to claim 9, wherein the preservative is benzyl alcohol, cresol, alkyl paraben or a mixture thereof, and the isotonic agent is a water-soluble inorganic salt and/or a sugar alcohol.

## Patentansprüche

1. Wässrige Formulierung eines humanen Follikel-stimulierenden Hormons (hFSH) umfassend eine therapeutisch wirksame Menge an hFSH und Glycin, Methionin, ein nicht-ionisches Tensid und einen Phosphatpuffer als Stabilisatoren.

2. Formulierung nach Anspruch 1, worin das hFSH ein natürlich vorkommendes hFSH oder ein rekombinantes FSH (rFSH) ist.

3. Formulierung nach Anspruch 1, worin das nicht-ionische Tensid aus der Gruppe bestehend aus einem nicht-ionischen Tensid auf Polysarbat-Basis, einem nicht-ionischen Tensid auf Poloxamer-Basis und einer beliebigen Kombination davon ausgewählt ist.

4. Formulierung nach Anspruch 3, worin das nicht-ionische Tensid Polysorbat 20 ist.

5. Formulierung nach Anspruch 1, worin Glycin in einer Menge von 01 bis 10% (WN) enthalten ist.

6. Formulierung nach Anspruch 1, worin Methionin in einer Menge von 0,01 bis 2,0 % (W/V) enthalten ist.

7. Formulierung nach Anspruch 1, worin das nicht-ionische Tensid in einer Menge von 0,001 bis 0,02 % (W/V) enthalten ist.

8. Formulierung nach Anspruch 1, worin der Phosphatpuffer eine Salzkonzentration von 1 mM bis 50 mM und einen pH-Wert von 6,5 bis 7,5 hat.

9. Formulierung nach Anspruch 1, worin die Formulierung weiterhin einen Konservierungsstoff und/oder ein isotonisches Mittel enthält.

10. Formulierung nach Anspruch 9, worin der Konservierungsstoff Benzylalkohol, Kresol, Alkylparaben oder eine Mischung davon und das isotonische Mittel ein wasserlösliches anorganisches Salz und/oder ein Zuckeralkohol ist.

## Revendications

1. Formulation aqueuse d'une hormone de stimulation folliculaire humaine (hFSH), comprenant une quantité thérapeutiquement efficace de hFSH, et de la glycine, de la méthionine, un tensioactif non ionique et un tampon de phosphate comme agents de stabilisation.

2. Formulation selon la revendication 1, dans laquelle la hFSH est une FSH d'origine naturelle ou une hFSH recombinant (rFSH).

3. Formulation selon la revendication 1, dans laquelle le tensioactif non ionique est choisi dans le groupe constitué par un tensioactif non ionique à base de polysorbate, un tensioactif non ionique à base de poloxamère et l'une quelconque de leurs combinaisons.

4. Formulation selon la revendication 3, dans laquelle le tensioactif non ionique est le polysorbate 20.

5. Formulation selon la revendication 1, dans laquelle la glycine est comprise en une quantité allant de 0,1 à 10 % (m/v).

6. Formulation selon la revendication 1, dans laquelle la méthionine est comprise en une quantité allant de 0,01 à 2,0% (m/v).

7. Formulation selon la revendication 1, dans laquelle le tensioactif non ionique est compris en une quantité allant de 0,001 à 0,02 % (m(v).

8. Formulation selon la revendication 1, dans laquelle le tampon de phosphate a une concentration en sel allant de 1 mM à 50 mM et un pH de 6,5 à 7,5.

9. Formulation selon la revendication 1, dans laquelle la formulation comprend en outre un conservateur et/ou un agent isotonique.

10. Formulation selon la revendication 9, dans laquelle le conservateur est l'alcool benzylique, le crésol, un alkylparabène ou l'un de leurs mélanges, et l'agent isotonique est un sel inorganique soluble dans l'eau et/ou un alcool de sucre.
